# EUROPEAN PATENT APPLICATION

(11) **EP 4 734 053 A1**
(43) Date of publication of application: **29.04.2026**
(21) Application number: 25211108.3
(22) Date of filing: 24.10.2025
(51) Int. Cl.: G06T 7/11

(54) **SYSTEM FOR PREDICTING THE ACCURACY OF SEGMENTATION LABELS USING MULTIPLE CONVOLUTIONAL NEURAL NETWORKS FROM MAGNETIC RESONANCE IMAGES**

(30) Priority: 25.10.2024 US 202418927288
(71) Applicant: Springbok, Inc., Charlottesville, Virginia 22902 (US)
(72) Inventor: RIEM, Lara, Charlottesville (US); FENG, Xue, Charlottesville (US); BLEMKER, Silvia, Charlottesville (US)
(74) Representative: Maiwald GmbH

(57) **Abstract**

The present disclosure relates to systems and methods for segmenting one or more regions of interest (ROI) in medical image data. These include segmenting a plurality of medical images by inputting the plurality of medical images into each of a plurality of trained convolutional neural networks (CNNs) to identify a group of the plurality of voxels belonging to one or more ROI; calculating a plurality of variables from each of the segmented plurality of medical images on a voxel-by-voxel basis or on a ROI-by-ROI basis; generating a segmentation accuracy score from the calculated variables; and outputting a label for the one or more ROI.

## Description

### Cross Reference to Related Applications

N/A

### Statement of Government Support

This invention was made with government support under R44AR078720 awarded by the National Institute of Health. The government has certain rights in the invention.

### Background

Image analysis of the musculoskeletal system is often performed manually, which is time consuming. Moreover, manual analysis leads to subjective results that vary based on who performs the analysis, the processing and visualization tools available, and imaging protocols used. Several techniques have been developed for automatic muscle segmentation, such as atlas-based and shape-based methods. Recently, deep convolutional neural network (DCNN) has been applied in many medical image segmentation tasks from MRI or computed tomography (CT) images.

However, these CNNs perform at varying levels depending on the muscle or region of interest being segmented. Thus, there is a need to improve segmentation accuracy of CNN outputs.

### Summary

The present disclosure provides systems and methods that overcome the aforementioned drawbacks by utilizing an artificial intelligence (AI) platform combining a plurality of models with different network parameters for increasing the accuracy of region of interest (ROI) labeling.

In one aspect of the present disclosure, a system for segmenting one or more ROIs in medical image data is described. The system comprises a memory configured to store instructions and a plurality of medical images of a subject, wherein the medical images comprise a plurality of pixels or voxels. The system includes a processor configured to access the memory, segment the plurality of medical images by inputting the plurality of medical images into each of a plurality of convolutional neural networks (CNNs) to identify a group of the plurality of pixels or voxels belonging to one or more ROI, calculate a plurality of variables from each of the segmented plurality of medical images on a pixel-by-pixel basis, a voxel-by-voxel basis, or a ROI-by-ROI basis, generate a segmentation accuracy score from the calculated variables, and output a label for the one or more ROI. The system further includes a display configured to display at least one of the segmentation accuracy score or the label.

In another aspect of the present disclosure, a method of segmenting one or more regions of interest in medical image data is presented. The method comprises accessing a plurality of medical images of a subject using a processor, the medical images comprising a plurality of pixels or voxels. The method further comprises segmenting the plurality of medical images using the processor by inputting the plurality of medical images into each of a plurality of convolutional neural networks (CNNs) to identify a group of the plurality of pixels or voxels belonging to one or more ROI, calculating a plurality of variables from each of the segmented plurality of medical images on a pixel-by-pixel basis, a voxel-by-voxel basis, or a ROI-by-ROI basis, generating a segmentation accuracy score from the calculated variables, and outputting a label for the one or more ROI.

These aspects are nonlimiting. Other aspects and features of the systems and methods described herein will be provided below.

### Brief Description of the Drawings

The foregoing features of embodiments will be more readily understood by reference to the following detailed description, taken with reference to the accompanying drawings, in which:
FIG. 1 is a schematic diagram of one non-limiting example of a framework for implementing a multi-model platform in accordance with the present disclosure.
FIG. 2 is a block diagram of an example ROI label prediction system.
FIG. 3 is a block diagram of example components that can implement the ROI label prediction system of FIG. 2.
FIG. 4A is a flow chart setting forth some non-limiting example steps of a process that can be implemented using a platform, for example, such as described with respect to FIG. 1.
FIG. 4B is a flow chart setting forth some non-limiting example steps of an implementation using a platform.
FIG. 5A is a schematic of a single model platform.
FIG. 5B is a schematic of a three-model platform, according to aspects of the present disclosure.
FIG. 6A shows a plot of the dice score (top) and volume error (bottom) comparing the combined 3-model platform and each individual single-model of the 3-model platform, according to aspects of the present disclosure.
FIG. 6B shows a plot of the dice score (top) and volume error (bottom) comparing the combined 3-model platform and each individual single-model of the 3-model platform, according to aspects of the present disclosure.
FIG. 7 is a series of plots of the model comparisons for predicting the AI's accuracy for the biceps femoris muscle.
FIG. 8A is a series of plot showing the dice score (top) and volume error (bottom) of a variety of muscles.
FIG. 8B is a table of dice scores for each of the muscles plotted in FIG. 8A.
FIG. 8C is a table of volume error (%) for each of the muscles plotted in FIG. 8A.
FIG. 9A is a model of labeled muscles using the old single model AI framework.
FIG. 9B is a model of labeled muscles using single model 1, according to aspects of the present disclosure.
FIG. 9C is a model of labeled muscles using the combined 3-model AI platform, according to aspects of the present disclosure.
FIG. 9D is a model of labeled muscles vetted by a skilled user.
FIG. 10A is a series of plots of dice scores (top) and volume error (bottom) comparing labels between the combined 3-model AI platform and those vetted by a skilled user.
FIG. 10B is a schematic of muscle label confidence based on the comparisons in FIG. 10A.
FIG. 11A is a model of labeled muscles using the old single model AI framework.
FIG. 11B is a model of labeled muscles using single model 1, according to aspects of the present disclosure.
FIG. 11C is a model of labeled muscles using the combined 3-model AI platform, according to aspects of the present disclosure.
FIG. 11D is a model of labeled muscles vetted by an experienced user.
FIG. 12A is a series of plots of dice scores (top) and volume error (bottom) comparing labels between the combined 3-model AI platform and those vetted by a skilled user.
FIG. 12B is a schematic of muscle label confidence based on the comparisons in FIG. 12A.

### Detailed Description

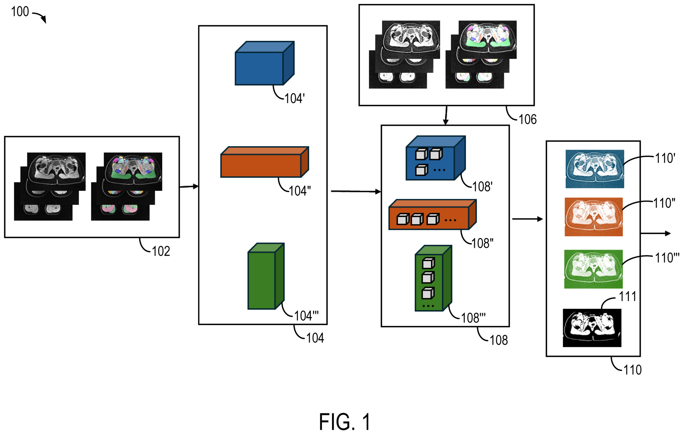

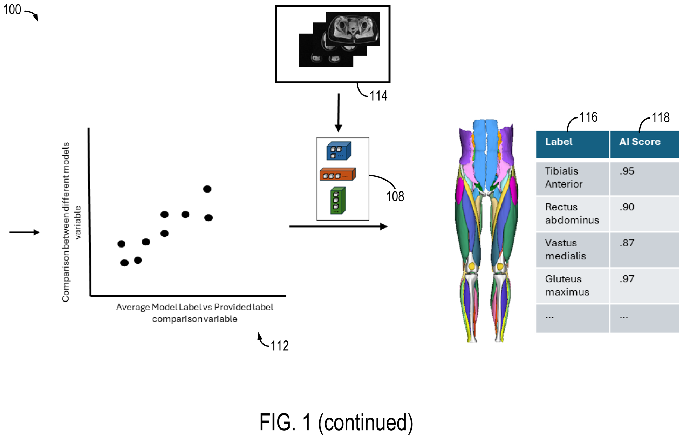

Referring now to Fig. 1, a schematic diagram of a non-limiting example of a framework 100 for implementing a multi-modal AI platform is provided. A plurality of medical images with segmented ROI 102 are used to train a plurality of different CNNs 104. For example, the ROI may include, but is not limited to, one or more muscles. In a non-limiting example, the medical images include magnetic resonance (MR) images. The schematic of FIG. 1 illustrates MR images, but is not intended to be limiting. In FIG. 1, three CNNs 104', 104", 104‴ are trained, however, the framework need not be limited to three CNNs. In a non-limiting example, the CNNs 104 differ in one or more parameters. For example, a parameter may include a network structure, one or more parameters during training, a training set, or one or more parameters during a deployment of the system. In a non-limiting example, the parameter may include a window size, learning rate, training input resolution, training dataset, or whether the training data is augmented.

An additional plurality of images with segmented regions of interest 106 are deployed on the trained CNNs 108. Outputs 110 from each trained CNN 108', 108", 108‴ are recorded. The outputs 110 include segmented outputs 110', 110", 110‴ as well as an averaged output 111 in which the probability for each ROI on each voxel across each trained CNN is averaged to find the final label.

The resultant segmentations 110 are examined for each ROI. The labeled ROI are compared across the trained CNNs using a plurality variables. In a non-limiting example, the variables include at least one of a median, a standard deviation, a label volume variation, a label dice, or a label probability between each of the plurality of trained CNNs. The error between the averaged CNN's 111 labels and the provided labels inputted with the images 106 is found using the different variables. The plurality of variables measured across the differing input images 106 are combined together per ROI. The comparison variables between the average model label and provided label is related to the comparison variable(s) across models as shown in plot 112. These metrices are related and may be used to define an AI prediction score as the error between AI label output and the desired label output.

A new plurality of medical images 114 can be deployed by the plurality of trained CNN(s) 108. The new plurality of medical images 114 are segmented to identify a group of the plurality of voxels belonging to one or more ROI. In a non-limiting example, the trained CNNs use a sliding window approach to segment the new plurality of medical images 114. Using the relation defined previously in the creation of plot 112, the final averaged model's 111 labels are used as the official AI label output 116, and the needed comparison variables between the trained CNNs are used to calculate a segmentation accuracy score 118 for each label (referred to as "AI Score" in FIG. 1). The segmentation accuracy score will relate how well or bad the AI predicts the segmentation. In a non-limiting example, the AI label output 116 and segmentation accuracy score 118 are presented to a user on a display.

As used herein, the term "CNN" may be interchangeable with "model" or "AI."

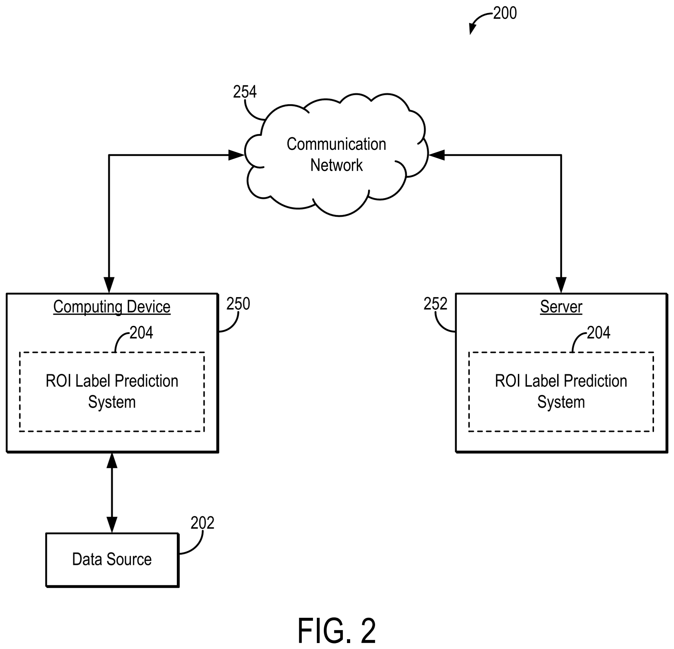

Referring now to FIG. 2, an example of a system 200 for generating ROI labels in accordance with some embodiments of the systems and methods described in the present disclosure is shown. As shown in FIG. 2, a computing device 250 can receive one or more types of medical image data (e.g., magnetic resonance (MR) images) from data source 202. In some embodiments, computing device 250 can execute at least a portion of a ROI label prediction system 204 to generate ROI labels from data received from the data source 202.

Additionally or alternatively, in some embodiments, the computing device 250 can communicate information about data received from the data source 202 to a server 252 over a communication network 254, which can execute at least a portion of the ROI label prediction system 204. In such embodiments, the server 252 can return information to the computing device 250 (and/or any other suitable computing device) indicative of an output of the ROI label prediction system 204.

In some embodiments, computing device 250 and/or server 252 can be any suitable computing device or combination of devices, such as a desktop computer, a laptop computer, a smartphone, a tablet computer, a wearable computer, a server computer, a virtual machine being executed by a physical computing device, and so on. The computing device 2550 and/or server 252 can also reconstruct images from the data.

In some embodiments, data source 202 can be any suitable source of data (e.g., measurement data, images reconstructed from measurement data, processed image data), such as an MR system or another computing device (e.g., a server storing measurement data, images reconstructed from measurement data, processed image data), and so on. In some embodiments, data source 202 can be local to computing device 250. For example, data source 202 can be incorporated with computing device 250 (e.g., computing device 250 can be configured as part of a device for measuring, recording, estimating, acquiring, or otherwise collecting or storing data). As another example, data source 202 can be connected to computing device 250 by a cable, a direct wireless link, and so on. Additionally or alternatively, in some embodiments, data source 202 can be located locally and/or remotely from computing device 250, and can communicate data to computing device 250 (and/or server 252) via a communication network (e.g., communication network 254).

In some embodiments, communication network 254 can be any suitable communication network or combination of communication networks. For example, communication network 254 can include a Wi-Fi network (which can include one or more wireless routers, one or more switches, etc.), a peer-to-peer network (e.g., a Bluetooth network), a cellular network (e.g., a 3G network, a 4G network, etc., complying with any suitable standard, such as CDMA, GSM, LTE, LTE Advanced, WiMAX, etc.), other types of wireless network, a wired network, and so on. In some embodiments, communication network 254 can be a local area network, a wide area network, a public network (e.g., the Internet), a private or semi-private network (e.g., a corporate or university intranet), any other suitable type of network, or any suitable combination of networks. Communications links shown in FIG. 2 can each be any suitable communications link or combination of communications links, such as wired links, fiber optic links, Wi-Fi links, Bluetooth links, cellular links, and so on.

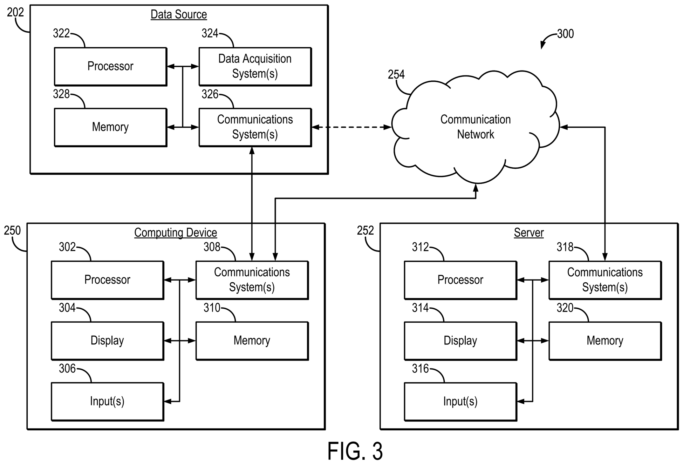

Referring now to FIG. 3, an example of hardware 300 that can be used to implement data source 202, computing device 250, and server 252 in accordance with some embodiments of the systems and methods described in the present disclosure is shown.

As shown in FIG. 3, in some embodiments, computing device 250 can include a processor 302, a display 304, one or more inputs 306, one or more communication systems 308, and/or memory 310. In some embodiments, processor 302 can be any suitable hardware processor or combination of processors, such as a central processing unit ("CPU"), a graphics processing unit ("GPU"), and so on. In some embodiments, display 304 can include any suitable display devices, such as a liquid crystal display ("LCD") screen, a light-emitting diode ("LED") display, an organic LED ("OLED") display, an electrophoretic display (e.g., an "e-ink" display), a computer monitor, a touchscreen, a television, and so on. In some embodiments, inputs 306 can include any suitable input devices and/or sensors that can be used to receive user input, such as a keyboard, a mouse, a touchscreen, a microphone, and so on.

In some embodiments, communications systems 308 can include any suitable hardware, firmware, and/or software for communicating information over communication network 254 and/or any other suitable communication networks. For example, communications systems 308 can include one or more transceivers, one or more communication chips and/or chip sets, and so on. In a more particular example, communications systems 308 can include hardware, firmware, and/or software that can be used to establish a Wi-Fi connection, a Bluetooth connection, a cellular connection, an Ethernet connection, and so on.

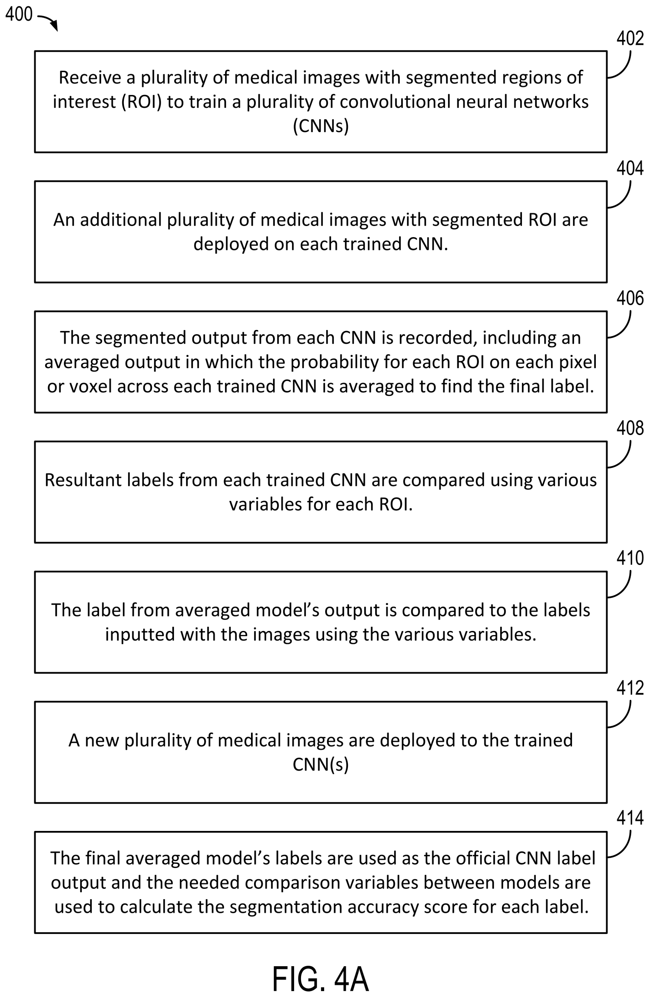

In some embodiments, memory 310 can include any suitable storage device or devices that can be used to store instructions, values, data, or the like, that can be used, for example, by processor 302 to present content using display 304, to communicate with server 252 via communications system(s) 308, and so on. Memory 310 can include any suitable volatile memory, non-volatile memory, storage, or any suitable combination thereof. For example, memory 310 can include random-access memory ("RAM"), read-only memory ("ROM"), electrically programmable ROM ("EPROM"), electrically erasable ROM ("EEPROM"), other forms of volatile memory, other forms of non-volatile memory, one or more forms of semi-volatile memory, one or more flash drives, one or more hard disks, one or more solid state drives, one or more optical drives, and so on. In some embodiments, memory 310 can have encoded thereon, or otherwise stored therein, a computer program for controlling operation of computing device 250. In such embodiments, processor 302 can execute at least a portion of the computer program to present content (e.g., images, user interfaces, graphics, tables), receive content from server 252, transmit information to server 252, and so on. For example, the processor 302 and the memory 310 can be configured to perform the methods described herein (e.g., the methods of FIGS. 4A-4B).

In some embodiments, server 252 can include a processor 312, a display 314, one or more inputs 316, one or more communications systems 318, and/or memory 320. In some embodiments, processor 312 can be any suitable hardware processor or combination of processors, such as a CPU, a GPU, and so on. In some embodiments, display 314 can include any suitable display devices, such as an LCD screen, LED display, OLED display, electrophoretic display, a computer monitor, a touchscreen, a television, and so on. In some embodiments, inputs 316 can include any suitable input devices and/or sensors that can be used to receive user input, such as a keyboard, a mouse, a touchscreen, a microphone, and so on.

In some embodiments, communications systems 318 can include any suitable hardware, firmware, and/or software for communicating information over communication network 254 and/or any other suitable communication networks. For example, communications systems 318 can include one or more transceivers, one or more communication chips and/or chip sets, and so on. In a more particular example, communications systems 318 can include hardware, firmware, and/or software that can be used to establish a Wi-Fi connection, a Bluetooth connection, a cellular connection, an Ethernet connection, and so on.

In some embodiments, memory 320 can include any suitable storage device or devices that can be used to store instructions, values, data, or the like, that can be used, for example, by processor 312 to present content using display 314, to communicate with one or more computing devices 250, and so on. Memory 320 can include any suitable volatile memory, non-volatile memory, storage, or any suitable combination thereof. For example, memory 320 can include RAM, ROM, EPROM, EEPROM, other types of volatile memory, other types of non-volatile memory, one or more types of semi-volatile memory, one or more flash drives, one or more hard disks, one or more solid state drives, one or more optical drives, and so on. In some embodiments, memory 320 can have encoded thereon a server program for controlling operation of server 252. In such embodiments, processor 312 can execute at least a portion of the server program to transmit information and/or content (e.g., data, images, a user interface) to one or more computing devices 250, receive information and/or content from one or more computing devices 250, receive instructions from one or more devices (e.g., a personal computer, a laptop computer, a tablet computer, a smartphone), and so on.

In some embodiments, the server 252 is configured to perform the methods described in the present disclosure. For example, the processor 312 and memory 320 can be configured to perform the methods described herein (e.g., the methods of FIGS. 4A-4B).

In some embodiments, data source 202 can include a processor 322, one or more data acquisition systems 324, one or more communications systems 326, and/or memory 328. In some embodiments, processor 322 can be any suitable hardware processor or combination of processors, such as a CPU, a GPU, and so on. In some embodiments, the one or more data acquisition systems 324 are generally configured to acquire data, images, or both, and can include medical imaging systems (e.g., MR system). Additionally or alternatively, in some embodiments, the one or more data acquisition systems 324 can include any suitable hardware, firmware, and/or software for coupling to and/or controlling operations of the medical imaging systems. In some embodiments, one or more portions of the data acquisition system(s) 324 can be removable and/or replaceable.

Note that, although not shown, data source 202 can include any suitable inputs and/or outputs. For example, data source 202 can include input devices and/or sensors that can be used to receive user input, such as a keyboard, a mouse, a touchscreen, a microphone, a trackpad, a trackball, and so on. As another example, data source 202 can include any suitable display devices, such as an LCD screen, an LED display, an OLED display, an electrophoretic display, a computer monitor, a touchscreen, a television, etc., one or more speakers, and so on.

In some embodiments, communications systems 326 can include any suitable hardware, firmware, and/or software for communicating information to computing device 250 (and, in some embodiments, over communication network 254 and/or any other suitable communication networks). For example, communications systems 326 can include one or more transceivers, one or more communication chips and/or chip sets, and so on. In a more particular example, communications systems 326 can include hardware, firmware, and/or software that can be used to establish a wired connection using any suitable port and/or communication standard (e.g., VGA, DVI video, USB, RS-232, etc.), Wi-Fi connection, a Bluetooth connection, a cellular connection, an Ethernet connection, and so on.

In some embodiments, memory 328 can include any suitable storage device or devices that can be used to store instructions, values, data, or the like, that can be used, for example, by processor 322 to control the one or more data acquisition systems 324, and/or receive data from the one or more data acquisition systems 324; to generate images from data; present content (e.g., data, images, a user interface) using a display; communicate with one or more computing devices 250; and so on. Memory 328 can include any suitable volatile memory, non-volatile memory, storage, or any suitable combination thereof. For example, memory 328 can include RAM, ROM, EPROM, EEPROM, other types of volatile memory, other types of non-volatile memory, one or more types of semi-volatile memory, one or more flash drives, one or more hard disks, one or more solid state drives, one or more optical drives, and so on. In some embodiments, memory 328 can have encoded thereon, or otherwise stored therein, a program for controlling operation of data source 202. In such embodiments, processor 322 can execute at least a portion of the program to generate images, transmit information and/or content (e.g., data, images, a user interface) to one or more computing devices 250, receive information and/or content from one or more computing devices 250, receive instructions from one or more devices (e.g., a personal computer, a laptop computer, a tablet computer, a smartphone, etc.), and so on.

In some embodiments, any suitable computer-readable media can be used for storing instructions for performing the functions and/or processes described herein. For example, in some embodiments, computer-readable media can be transitory or non-transitory. For example, non-transitory computer-readable media can include media such as magnetic media (e.g., hard disks, floppy disks), optical media (e.g., compact discs, digital video discs, Blu-ray discs), semiconductor media (e.g., RAM, flash memory, EPROM, EEPROM), any suitable media that is not fleeting or devoid of any semblance of permanence during transmission, and/or any suitable tangible media. As another example, transitory computer-readable media can include signals on networks, in wires, conductors, optical fibers, circuits, or any suitable media that is fleeting and devoid of any semblance of permanence during transmission, and/or any suitable intangible media.

As used herein in the context of computer implementation, unless otherwise specified or limited, the terms "component," "system," "module," "framework," and the like are intended to encompass part or all of computer-related systems that include hardware, software, a combination of hardware and software, or software in execution. For example, a component may be, but is not limited to being, a processor device, a process being executed (or executable) by a processor device, an object, an executable, a thread of execution, a computer program, or a computer. By way of illustration, both an application running on a computer and the computer can be a component. One or more components (or system, module, and so on) may reside within a process or thread of execution, may be localized on one computer, may be distributed between two or more computers or other processor devices, or may be included within another component (or system, module, and so on).

Referring now to FIG. 4A, a flowchart 400 is provided for one, non-limiting example process that can be carried out using a platform, for example, such as the framework 100 described with respect to Fig. 1. The process 400 begins at process block 402 whereby a system receives a plurality of medical images with segmented ROI to train a plurality of CNNs. In a non-limiting example, the medical images include MR images. As previously described, each of the plurality of CNNs may vary in parameters, such as, but not limited to a network structure, one or more network parameters during training, a training set, or one or more parameters during a deployment. As previously described, the parameters may include a window size, learning rate, training input resolution, training dataset, or whether the training data is augmented. At step 404, an additional plurality of images with segmented ROI are deployed on each trained CNN of the plurality of trained CNNs. At step 406, the segmented output from each of the trained CNNs is recorded, including an averaged output in which the probability for each ROI on each voxel across each trained CNN is average to find the final label. At step 408 the resultant labels from each of the trained CNNs are compared using a plurality of variables for each ROI. As described previously, the plurality of variable may include at least one of a median, a standard deviation, a label volume variation, a label dice, a or a label probability between each of the plurality of trained CNNs. For example, the different models' labels are compared for each ROI. An example comparison variable could be a dice score between labels or a volume error. At step 410, the label from the averaged model's outputs is compared to the provided labels inputted with the input images using different variables and the comparison variables of step 408 that most accurately predict the averaged model's labels are identified. At step 412, a new plurality of images is deployed to the trained CNNs. At step 414, the final averaged model's labels are used as the official CNN label output and the needed comparison variables between models are used to calculate a segmentation accuracy score for each label. The needed comparison variables - or the comparison variables used to predict the combined label vs what would be correct when implemented - refer to the comparison variable with the best correlation between itself and the combined label vs inputted label in step 414.

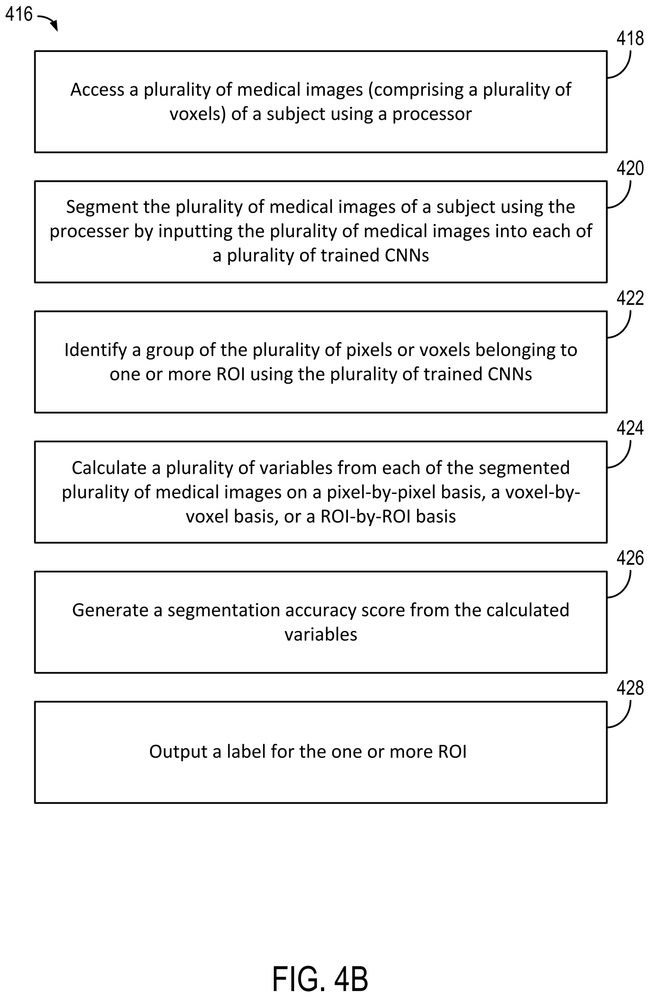

Referring now to FIG. 4B, a process 416 of generating output labels is described. At step 418, a plurality of medical images of a subject comprising a plurality of pixels or voxels is accessed by a processor. Ina non-limiting example, the medical images may be MR images. At step 420, the plurality of medical images is segmented by inputting the plurality of medical images into each of a plurality if trained CNNs. At step 422, a group of the plurality of pixels or voxels belonging to one or more ROI are identified using the plurality of trained CNNs. At step 424, a plurality of variables from each of the segmented plurality of medical images are calculated on a pixel-by-pixel, voxel-by-voxel, or ROI-by-ROI basis. At step 426, a segmentation accuracy score is generated from the calculated variables, as previously described. Finally at step 428, one or more ROI labels are output.

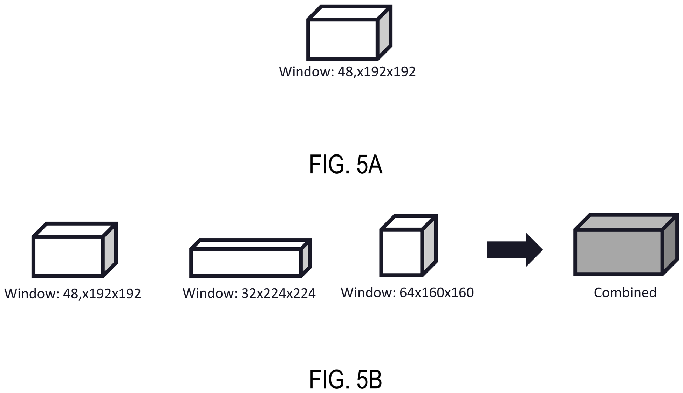

The systems and methods described above provide clinical utility not realized by traditional systems and methods. In particular, referring now to FIGS. 5A-5B a comparison of a previous single-model platform (FIG. 5A) is compared with the new 3-model AI platform (FIG. 5B) as described herein. In a non-limiting example, the 3-model platform is a combination of three single models with varying window sizes.

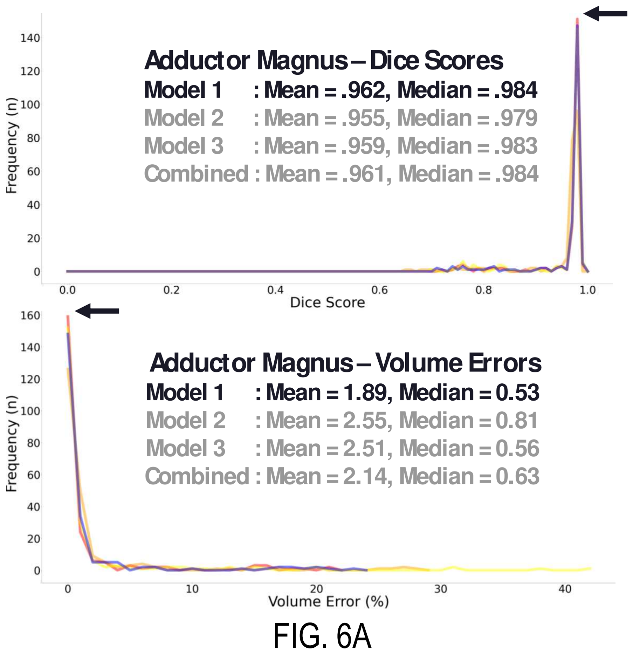

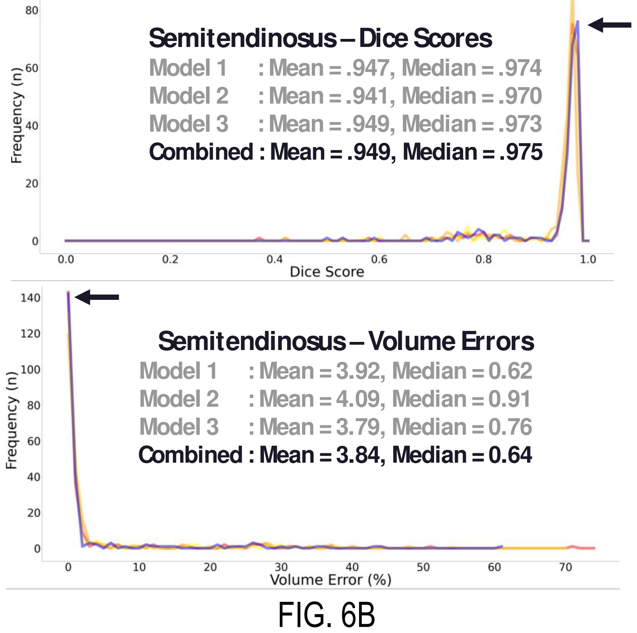

FIGS. 6A-6B illustrate that the different single models or the combined 3-model platform are better suited for identifying different muscles or regions of interest. FIG. 6A shows the dice score (top) and volume error (%, bottom), where single model 1 performs better than the single model 2, single model 3, or the combined 3-model platform for the Adductor Magnus muscle. Likewise, FIG. 6B shows the dice score (top) and volume error (%, bottom), where the combined 3-model platform performs better than single model 1, single model 2, or single model 3 for the Semitendinosus muscle.

In one example, the single models (1, 2, 3) and the combined 3-model platform were validated with various tomography scans (n=109). In a preferred example, the tomography scans are magnetic resonance (MR) images. The scans were input into the three single models and the combined 3-model platform and various metrics were obtained regarding the models' confidence for a given ROI: Average Label Probability for each single model 1-3, Variability in Label Probability between models, Average Dice Score between the 3 labels from the three models, and Average Volume Error between the 3 labels form the three models. From the labels for each of the three models, label maps were created and various metrics were obtained from each ROI: Dice Scores of Vetted vs Labels from Models 1, 2, and 3, Dice Scores of Vetted vs Label from the combined 3-model platform, Volume Error of Vetted vs Labels from Models 1, 2, and 3, and Volume Error of Vetted vs Label from the combined 3-model platform.

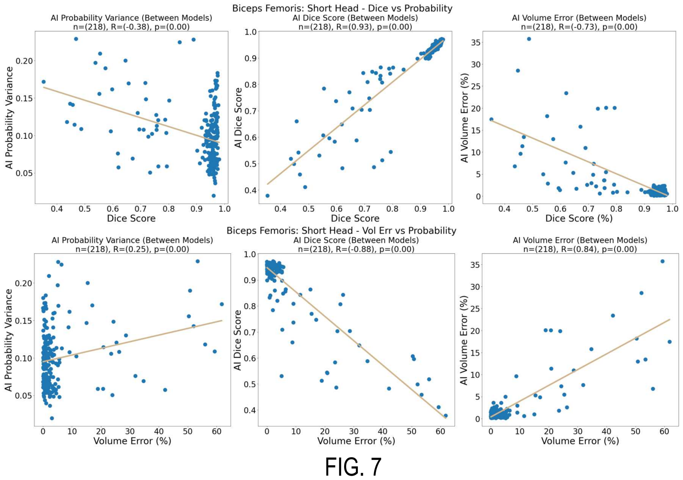

Referring to FIG. 7, a series of plots showing an example where the AI Dice score between models strongly predicts the final label to vetted dice score as well as predicting volume error for the biceps femoris muscle.

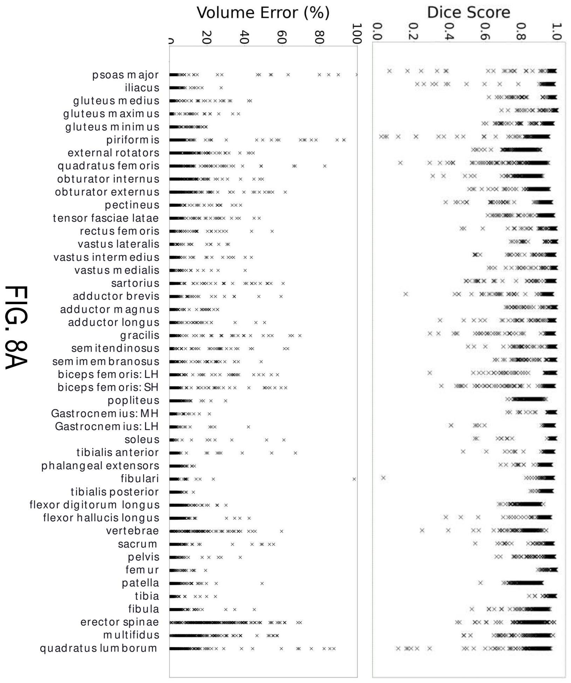

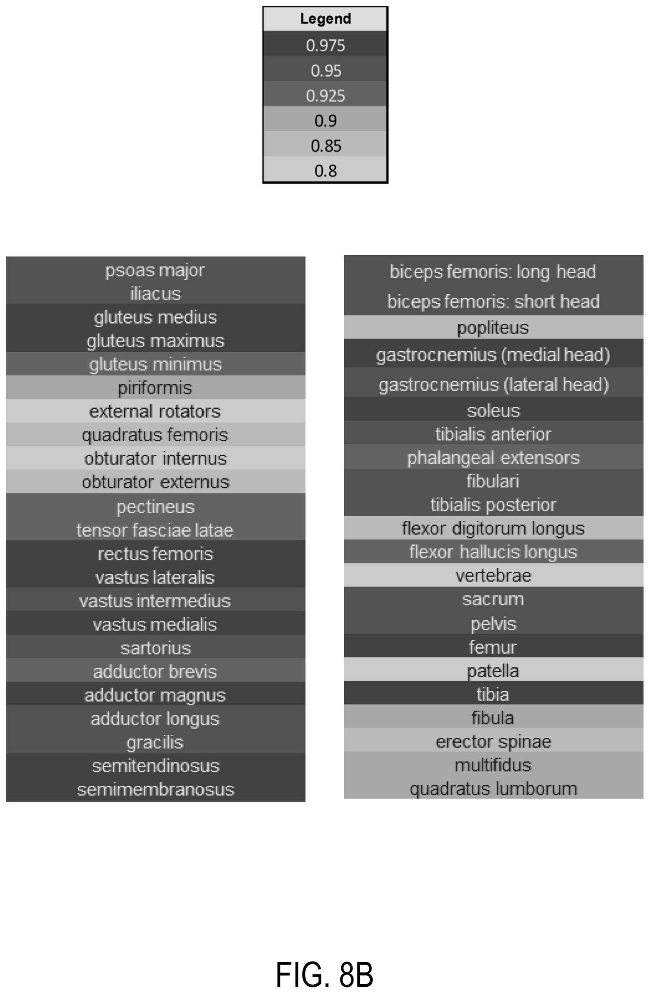

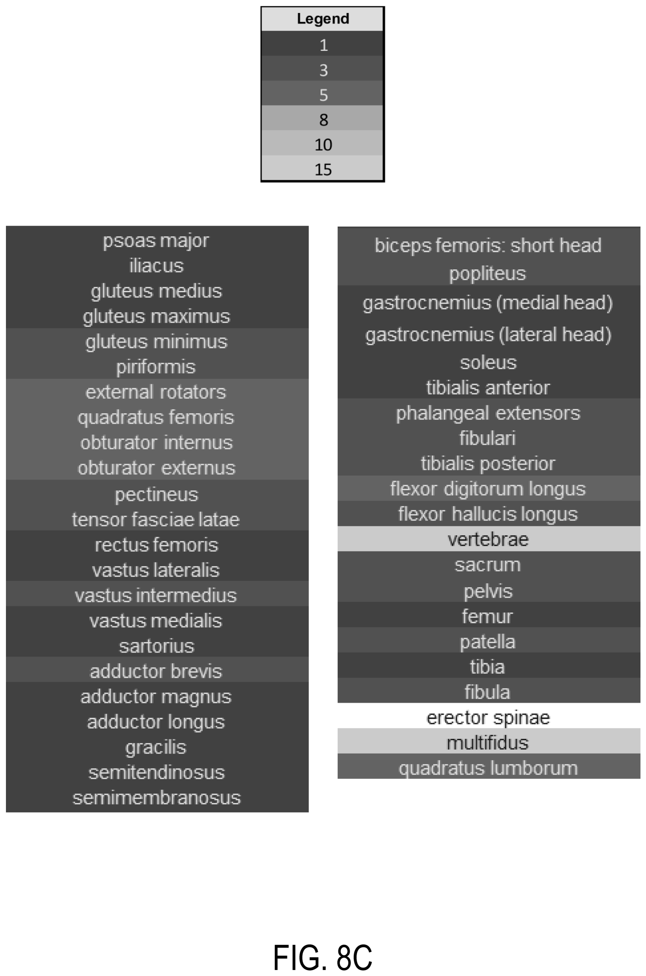

FIGS. 8A-8C illustrates the performance of the AI of a variety of muscles and their respective Dice Scores and Volume Error (%).

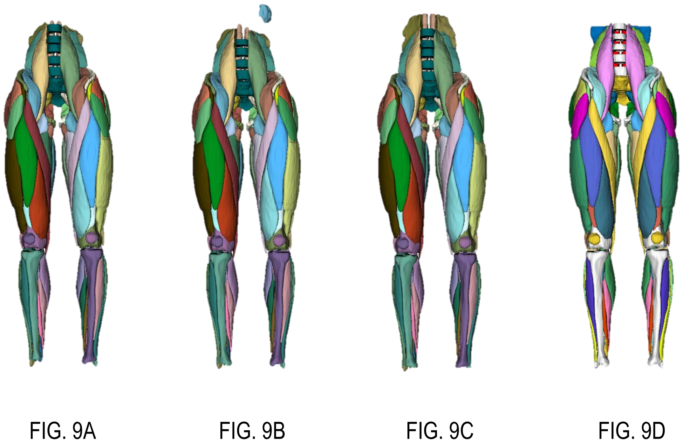

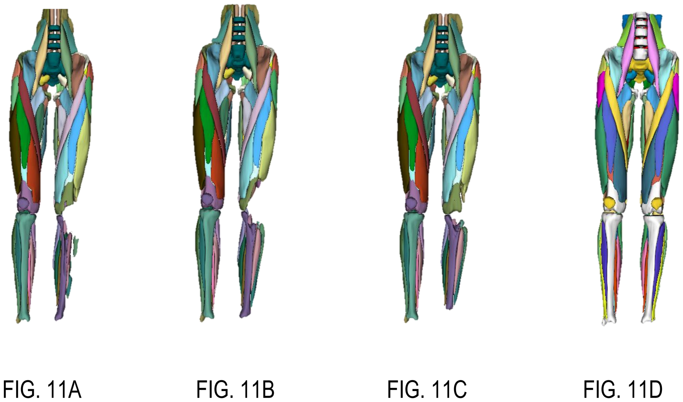

FIGS. 9A-9D and 11A-11D illustrate a model of labeled muscles using the previous single AI model (FIGS. 9A, 11A), the single model 1 of the AI platform described herein (FIGS. 9B, 11B), the combined 3-model AI platform (FIGS. 9C, 11C), and vetted by a skilled user (FIGS. 9D, 11D).

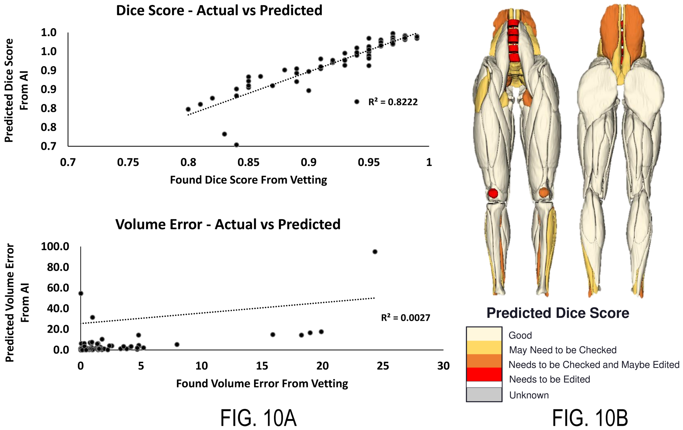

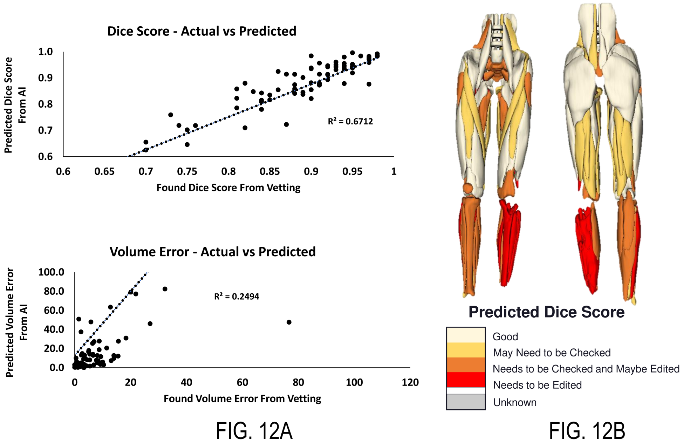

Referring to FIGS. 10A-10B and FIGS. 12A-12B, the comparison of the label Dice scores and Volume Error between the combined 3-model AI platform and the vetted model produce a heat map indicating the accuracy of the combined 3-model AI platform predictions.

As used in this specification and the claims, the singular forms "a," "an," and "the" include plural forms unless the context clearly dictates otherwise.

As used herein, "about", "approximately," "substantially," and "significantly" will be understood by persons of ordinary skill in the art and will vary to some extent on the context in which they are used. If there are uses of the term which are not clear to persons of ordinary skill in the art given the context in which it is used, "about" and "approximately" will mean up to plus or minus 10% of the particular term and "substantially" and "significantly" will mean more than plus or minus 10% of the particular term.

As used herein, the terms "include" and "including" have the same meaning as the terms "comprise" and "comprising." The terms "comprise" and "comprising" should be interpreted as being "open" transitional terms that permit the inclusion of additional components further to those components recited in the claims. The terms "consist" and "consisting of" should be interpreted as being "closed" transitional terms that do not permit the inclusion of additional components other than the components recited in the claims. The term "consisting essentially of" should be interpreted to be partially closed and allowing the inclusion only of additional components that do not fundamentally alter the nature of the claimed subject matter.

The phrase "such as" should be interpreted as "for example, including." Moreover, the use of any and all exemplary language, including but not limited to "such as", is intended merely to better illuminate the invention and does not pose a limitation on the scope of the invention unless otherwise claimed.

Furthermore, in those instances where a convention analogous to "at least one of A, B and C, etc." is used, in general such a construction is intended in the sense of one having ordinary skill in the art would understand the convention (e.g., "a system having at least one of A, B and C" would include but not be limited to systems that have A alone, B alone, C alone, A and B together, A and C together, B and C together, and/or A, B, and C together.). It will be further understood by those within the art that virtually any disjunctive word and/or phrase presenting two or more alternative terms, whether in the description or figures, should be understood to contemplate the possibilities of including one of the terms, either of the terms, or both terms. For example, the phrase "A or B" will be understood to include the possibilities of "A" or "B" or "A and B."

All language such as "up to," "at least," "greater than," "less than," and the like, include the number recited and refer to ranges which can subsequently be broken down into ranges and subranges. A range includes each individual member. Thus, for example, a group having 1-3 members refers to groups having 1, 2, or 3 members. Similarly, a group having 6 members refers to groups having 1, 2, 3, 4, or 6 members, and so forth.

The modal verb "may" refers to the preferred use or selection of one or more options or choices among the several described embodiments or features contained within the same. Where no options or choices are disclosed regarding a particular embodiment or feature contained in the same, the modal verb "may" refers to an affirmative act regarding how to make or use an aspect of a described embodiment or feature contained in the same, or a definitive decision to use a specific skill regarding a described embodiment or feature contained in the same. In this latter context, the modal verb "may" has the same meaning and connotation as the auxiliary verb "can."

## Claims

1. A system for segmenting one or more regions of interest (ROI) in medical image data, comprising:
a memory configured to store instructions and a plurality of medical images of a subject, wherein the medical images comprise a plurality of pixels or voxels;
a processor configured to:
access the memory;
segment the plurality of medical images by inputting the plurality of medical images into each of a plurality of trained convolutional neural networks (CNNs) to identify a group of the plurality of pixels or voxels belonging to one or more ROI;
calculate a plurality of variables from each of the segmented plurality of medical images on a pixel-by-pixel basis, a voxel-by-voxel basis, or a ROI-by-ROI basis;
generate a segmentation accuracy score from the plurality of calculated variables;
output a label for the one or more ROI; and
a display configured to display at least one of the segmentation accuracy score or the label.

2. The system of claim 1, wherein generating the segmentation accuracy score includes averaging the plurality of variables.

3. The system of claim 2, wherein each of the plurality of trained CNNs vary in at least one of a network structure, one or more network parameters during training, a training set, or one or more parameters during a deployment.

4. The system of claim 3, wherein the trained CNNs use a sliding window approach to segment the plurality of medical images during a deployment.

5. The system of claim 1, wherein the plurality of variables includes at least one of a median, a standard deviation, a label volume variation, a label dice, or a label probability between each of the plurality of trained CNNs.

6. The system of claim 1, wherein each of the plurality of trained CNNs are trained using labeled medical images.

7. The system of claim 1, wherein the one or more ROI includes one or more muscles.

8. The system of claim 1, wherein the plurality of medical images includes magnetic resonance (MR) images.

9. A method of segmenting one or more regions of interest in medical image data, comprising:
accessing a plurality of medical images of a subject using a processor, the medical images comprising a plurality of pixels or voxels;
segmenting the plurality of medical images using the processor by inputting the plurality of medical images into each of a plurality of trained convolutional neural networks (CNNs) to identify a group of the plurality of pixels or voxels belonging to one or more regions of interest (ROI);
calculating a plurality of variables from each of the segmented plurality of medical images on a pixel-by-pixel basis, a voxel-by-voxel basis, or a ROI-by-ROI basis;
generating a segmentation accuracy score from the plurality of calculated variables; and
outputting a label for the one or more ROI.

10. The method of claim 9, wherein generating the segmentation accuracy score includes averaging the plurality of variables.

11. The method of claim 10, wherein each of the plurality of CNNs vary in at least one of a network structure, one or more network parameters during training, a training set, or one or more parameters during a deployment.

12. The method of claim 11, wherein the trained CNNs use a sliding window approach to segment the plurality of medical images during a deployment.

13. The method of claim 9, wherein the plurality of variables includes at least one of a median, a standard deviation, a label volume variation, a label dice, or a label probability between each of the plurality of trained CNNs.

14. The method of claim 9, wherein each of the plurality of trained CNNs are trained using labeled medical images.

15. The method of claim 9, wherein the one or more ROI includes one or more muscles.

16. The method of claim 9, wherein the plurality of medical images includes magnetic resonance (MR) images.
